# FASCICULE DE BREVET EUROPEEN

(11) **EP 1 844 330 B1**
(45) Date de publication et mention de la délivrance du brevet: **23.03.2016**
(21) Numéro de dépôt: 06709209.8
(22) Date de dépôt: 31.01.2006
(51) Int. Cl.: G01N 33/542, G01N 33/24

(54) **PROCEDE DE DETERMINATION DE LA REACTIVITE BIOGEOCHIMIQUE D'UN ECHANTILLON AQUEUX DE MATIERE ORGANIQUE**
VERFAHREN ZUR BESTIMMUNG DER BIOGEOCHEMISCHEN REAKTIVITÄT EINER WÄSSERIGEN PROBE VON ORGANISCHEM MATERIAL
METHOD FOR DETERMINING THE BIOGEOCHEMICAL REACTIVITY OF AN ORGANIC AQUEOUS SAMPLE

(30) Priorité: 31.01.2005 FR 0500957
(43) Date de publication de la demande: 17.10.2007
(73) Titulaire: INSTITUT NATIONAL DE LA RECHERCHE AGRONOMIQUE (INRA), 75338 Paris Cédex 07 (FR); Université de Provence - Aix Marseille 1, 13331 Marseille (FR); Université De Reims Champagne-Ardenne, 51097 Reims (FR)
(72) Inventeur: DUDAL, Yves, 34730 Prades-Le-Lez (FR); GUILLON, Emmanuel, 51530 Dizy (FR); DUPONT, Laurent, 51100 Reims (FR); QUIQUAMPOIX, Hervé, 34150 La Boissiere (FR); BOUDENNE, Jean-Luc, 13009 Marseille (FR)
(74) Mandataire: Colombet, Alain André
(86) Numéro de dépôt international: PCT/FR2006/000215
(87) Numéro de publication internationale: WO 2006/079733

(56) Documents cités:
- WO-A-96/15448
- FANG FENG ET AL: "A spectrofluorimetric study of the binding of carbofuran, carbaryl, and aldicarb with dissolved organic matter" ANALYTICA CHIMICA ACTA, vol. 373, no. 2-3, 11 novembre 1998 (1998-11-11), pages 139-151, XP002358672 ISSN: 0003-2670 cité dans la demande
- KLAPPER LISA ET AL: "Fulvic acid oxidation state detection using fluorescence spectroscopy" ENVIRONMENTAL SCIENCE AND TECHNOLOGY, vol. 36, no. 14, 15 juillet 2002 (2002-07-15), pages 3170-3175, XP002358673 ISSN: 0013-936X
- SENESI N: "MOLECULAR AND QUANTITATIVE ASPECTS OF THE CHEMISTRY OF FULVIC ACID AND ITS INTERACTIONS WITH METAL IONS AND ORGANIC CHEMICALS PART II. THE FLUORESCENCE SPECTROSCOPY APPROACH" ANALYTICA CHIMICA ACTA, vol. 232, no. 1, 1990, pages 77-106, XP002358674 ISSN: 0003-2670

## Description

L'invention concerne un procédé de détermination de la réactivité biogéochimique d'échantillons aqueux de matières organiques, notamment aux fins d'en évaluer l'écotoxicité.

On estime qu'en France, 250 millions de tonnes de déchets agricoles et 10,5 millions de tonnes de déchets industriels sont recyclés chaque année par épandage au sol.

Cette pratique soulève la question du devenir des matières organiques contenues dans ces déchets dans les sols, de leur capacité à atteindre les nappes phréatiques, de leur influence sur les propriétés et fonctions des sols et des modifications biogéochimiques qu'elles impliquent, notamment en terme de pollution.

Il est fait généralement appel à des techniques d'analyses simples, notamment de la composition (carbone, azote, phosphore) des matières organiques présentes dans ces déchets, pour déterminer la quantité de déchets qu'il est possible d'épandre. Ces techniques ne permettent pas d'évaluer la réactivité des déchets dans le sol et, par conséquent, ne donnent pas d'indications sur leur durée de vie dans le sol, leur capacité de transfert vers la nappe phréatique et leurs impacts environnementaux potentiels, notamment en terme de mobilisation de micropolluants présents, métalliques et/ou organiques.

Fang et al. (1998) Analytica Chiica Acta 373:139-151 décrit l'étude de la liaison, par spectrofluorométrie, de trois pesticides avec de l'acide humique et des échantillons de sol.

Klapper et al. (2002) Environ. Sci. Technol. 36:3170-3175 décrit l'utilisation de la spectroscopie à fluorescence 3D pour évaluer l'état d'oxydation de substances humiques, par observation de la fluorescence naturelle de leurs résidus quinones.

Senesi et al. (1990) Analytica Chimica Acta 232:77-106 décrit l'étude des interactions de l'acide fulvique avec des ions métalliques et des composés organiques par spectrofluorométrie.

Il existe donc un besoin pour un test, de préférence simple et rapide, qui permette d'évaluer quantitativement le devenir de ces déchets dans les sols et les impacts environnementaux potentiels.

La présente invention répond à ce besoin, au moyen d'un test sur microplaque utilisant une lecture de fluorescence, qui permet de quantifier de manière routinière la réactivité d'un échantillon aqueux de matière organique, avec des composants du sol.

L'invention a donc pour objet un procédé de détermination de la réactivité biogéochimique d'un échantillon aqueux de matière organique, dans lequel le devenir de ladite matière organique un sol est évalué, comprenant :
a. le mélange de l'échantillon avec un composant d'un sol avec lequel l'échantillon est susceptible de réagir, ledit composant d'un sol étant un microorganisme; et
b. l'analyse en microplaque de la fluorescence émise par ledit mélange en présence d'un révélateur rédox fluorescent sensible au catabolisme de la matière organique de l'échantillon;
une variation de fluorescence, par rapport à la fluorescence émise par le composant du sol en l'absence de l'échantillon ou par l'échantillon en l'absence du composant du sol, étant indicatrice de la capacité de l'échantillon à être dégradé par un microorganisme du sol.

La demande décrit également un procédé de détermination de la réactivité biogéochimique d'un échantillon aqueux de matière organique, comprenant :
a. le mélange de l'échantillon avec un sol sec ou un composant d'un sol avec lequel l'échantillon est susceptible de réagir, puis
b. lorsque l'échantillon est mélangé avec un sol sec, la récupération de la phase liquide ; et
c. l'analyse en microplaque de la fluorescence émise par ledit mélange ou ladite phase liquide ;
une variation de fluorescence, par rapport à la fluorescence émise par le composant du sol en l'absence de l'échantillon ou par l'échantillon en l'absence du composant du sol, étant indicatrice de la réactivité de l'échantillon.

L'invention a également pour objet un kit pour la mise en oeuvre du procédé selon l'invention.

### Définitions :

L'expression « échantillon aqueux de matière organique » regroupe notamment des déchets ou produits fermentescibles liquides aqueux, tels que de boues de stations d'épuration ou des résidus ou effluents agro-alimentaires. Sont également inclues des eaux de surface, telles que des eaux de lacs, de sols, ou de rivières, des eaux marines ou des eaux souterraines, ou encore des extraits aqueux d'un sol ou de sols. Dans le cas d'un extrait aqueux de sol ou d'un déchet organique biphasique, on effectue une extraction à l'eau (typiquement une masse de sol ou de déchet pour deux volumes d'eau) et une centrifugation (par exemple au moins 5 minutes à 5000 rpm) puis une filtration de l'échantillon aqueux (par exemple, avec un filtre de 0,45 µm). Dans le cas d'un échantillon d'eau ou de déchet liquide (avec ou sans dilution préalable), une filtration simple peut être réalisée. De préférence, les échantillons aqueux de matière organique ne comprennent pas ou ne sont pas constitués d'acide fulvique ou d'acide humique.

Le terme « réactivité biogéochimique » fait référence à la capacité de l'échantillon de réagir avec certains composants du sol, tels que des métaux ou des microorganismes, ou avec un sol sec.

Plus particulièrement, le procédé décrit ici permet de quantifier la réactivité de l'échantillon testé avec un sol sec ou un composant d'un sol, ledit composant étant choisi parmi une exoenzyme, un microorganisme, un cation métallique et un micropolluant organique. De préférence, le sol sec ou le composant d'un sol ne comprennent pas ou ne sont pas constitués d'acide fulvique ou d'acide humique.

Ces composants sont également désignés ici « composants ou sondes biogéochimiques ».

En particulier, le procédé décrit ici est destiné à évaluer les interactions de la matière organique, dont provient l'échantillon aqueux de matière organique, avec un sol, soit directement à partir d'un échantillon de ce sol, soit à partir d'éléments représentatifs des sols, tels que les composants ou sondes biogéochimiques cités ci-dessus. Plus particulièrement, le procédé décrit ici est destiné à évaluer le devenir de ladite matière organique, ou d'associations de ladite matière organique avec certains éléments du sol, dans le sol en question.

### Format du test :

Le procédé développé en microplaque permet de travailler avec de faibles volumes, et permet de tester de nombreux échantillons rapidement.

De préférence, le volume de liquide déposé dans le puits de la microplaque est inférieur à environ 1 ml, de préférence inférieur à environ 500 µl, de préférence encore compris entre environ 200 et environ 400 µl. Le mélange de l'échantillon avec le composant du sol est réalisé soit directement dans les puits de la microplaque, soit préalablement. Lorsque le composant du sol est solide, le mélange avec l'échantillon est réalisé en dehors de la microplaque, puis généralement centrifugé, pour récupérer la phase liquide qui est déposée enfin dans les puits de la microplaque. Dans le procédé décrit ici, la « phase liquide » désigne donc la phase liquide de l'échantillon aqueux de matière organique qui a été mise en contact avec le sol sec.

La microplaque (typiquement, mais de manière non-obligatoire, à 96 puits) est choisie en une matière à auto-fluorescence minimale. Il peut s'agir par exemple d'une microplaque en polypropylène blanc et opaque.

Plusieurs fonctionnalités, à savoir plusieurs réactivités, peuvent être testées sur la même microplaque.

Des puits de calibration sont de manière avantageuse prévus, pour quantifier les réponses obtenues par les échantillons testés.

Le procédé décrit ici peut ainsi se présenter sous la forme d'un test multiformat, comprenant le mélange de l'échantillon séparément avec plusieurs sols secs ou composants du sol, et l'analyse de la fluorescence sur la même microplaque.

Plusieurs échantillons peuvent en outre être testés sur la même microplaque.

L'analyse de la fluorescence émise peut être réalisée simultanément pour l'ensemble des puits de la microplaque, ou, selon la nature du composant du sol, l'on peut analyser une partie de la microplaque, puis une autre plus tard, voire quelques heures ou jours après.

La lecture de fluorescence peut être réalisée par spectrofluométrie à l'aide d'un spectrofluorimètre (par exemple le modèle Perkin Elmer LS55) équipé d'un lecteur de microplaque.

Il est particulièrement avantageux de tester sur la même microplaque les cinq réactivités décrites plus en détails ci-dessous, à savoir tester sur la même microplaque la réactivité d'un échantillon avec un sol sec, une exoenzyme, un microorganisme, un cation métallique et un micropolluent organique. Ce test multiple, à détection unique (par fluorescence), permet d'évaluer facilement, et de manière quantitative, le devenir des échantillons aqueux de matière organique dans les sols, et les impacts environnementaux potentiels.

### Réactivité avec un sol sec :

L'échantillon à tester peut être mis en contact avec un sol sec. Par « sol sec », on entend un échantillon de sol à base de matière solide minérale et/ou organique, de préférence séché à environ 100-120°C, puis tamisé. Par exemple, on procède à un séchage à 105°C pendant 24 heures puis tamisage à 2 mm. On parle aussi ici de « surface solide ».

Dans ce cas, l'échantillon est mélangé avec le sol sec, la matière organique de l'échantillon s'adsorbe sur ledit sol sec puis la phase liquide est récupérée par centrifugation ou filtration sous vide. On analyse alors la diminution de la fluorescence émise par la phase liquide, du fait qu'une partie de la matière organique est restée adsorbée sur le sol sec, par rapport à la fluorescence émise par l'échantillon liquide qui n'a pas été en contact avec le sol sec. La contribution à la fluorescence du sol sec mélangé à un tampon aqueux est prise en compte dans chaque lecture.

Un exemple (hors invention) est décrit ci-après. Dans cet exemple, la réaction d'adsorption est réalisée dans un tampon, le MES (acide 2[N-Morpholino] éthanesulfonique), pH 6,3 en mélangeant l'échantillon au tampon dans un rapport compris entre 1/1 et 1/5 (1ml d'échantillon + 250 µl de tampon + 5 à 200 mg de surface). Ce mélange est mis en contact avec la matière solide pendant 24 heures dans des tubes eppendorf (1,5 ml). Différentes quantités de matière solide sont utilisées pour une même quantité d'échantillon liquide (4, 10, 20, 50, 75, 100 et 150 mg pour 1 ml). Pour chaque mesure, un second tube est nécessaire (même masse de surface + 1 ml d'eau + 250 µl de tampon MES) afin de mesurer la contribution à la fluorescence de l'extrait aqueux de quantité de matière solide donnée. Enfin, un contrôle permet de mesurer la contribution à la fluorescence de l'échantillon (1 ml d'échantillon liquide + 250 µl de tampon MES) afin de quantifier les contributions de fluorescence de chacune de ces deux composantes. Les 15 tubes sont placés à l'obscurité et à température constante (entre 20 et 25°C) pendant 24h sur un agitateur (environ 200 rpm). Le surnageant de chaque tube est ensuite récupéré après centrifugation (10 000 rpm, 5 minutes) et 270 µl de ce dernier sont déposés dans des puits de la microplaque.

La détection s'effectue de manière avantageuse aux longueurs d'onde d'excitation et d'émission propres à la matière organique soluble de l'échantillon, préalablement déterminées par un scan 3D sur le spectrofluorimètre. Néanmoins de manière générale, les coordonnées suivantes - longueur d'onde d'excitation λₑₓ 330 nm et longueur d'onde d'émission λₑₘ 440 nm- conviennent à la majorité des échantillons et permettent de minimiser l'autofluorescence de la.plaque, maximale pour les faibles longueur d'onde d'excitation. L'intensité de fluorescence (F) est lue par le lecteur de microplaque pour chacun des 15 puits.

Les lectures brutes servent à calculer le pourcentage de matière organique resté adsorbé sur la surface pour chaque quantité de surface différente selon l'équation suivante : [F(mat.org.) + F(surf) - F(surnageant)] / F(mat.org.).

L'interprétation peut se faire de deux manières en fonction de l'utilisation ou non d'une analyse mathématique. Premièrement, l'interprétation peut être réalisée par simple calcul du pourcentage de l'échantillon adsorbé, qui peut être ainsi comparé à d'autres échantillons ou fournir un ordre de grandeur de la réactivité entre l'échantillon et le sol. La lecture simple peut se faire sur la masse de sol à ajouter pour atténuer le signal de fluorescence de 50% (équivalent LD50) avec une grille de lecture en trois niveaux: moins de 20 mg (forte adsorption), entre 20 et 100 mg (adsorption moyenne), plus de 100 mg (faible adsorption) pour 1 ml d'échantillon. Deuxièmement, l'interprétation des résultats peut être réalisée à l'aide du modèle simple affinité/saturation (de type Michaelis-Menten ou Langmuir) qui fournit un paramètre quantitatif: l'affinité entre les matières organiques solubles et le sol. L'affinité représente la masse de sol à rajouter pour obtenir la demi-saturation, donc plus la valeur est faible, plus l'affinité est forte.

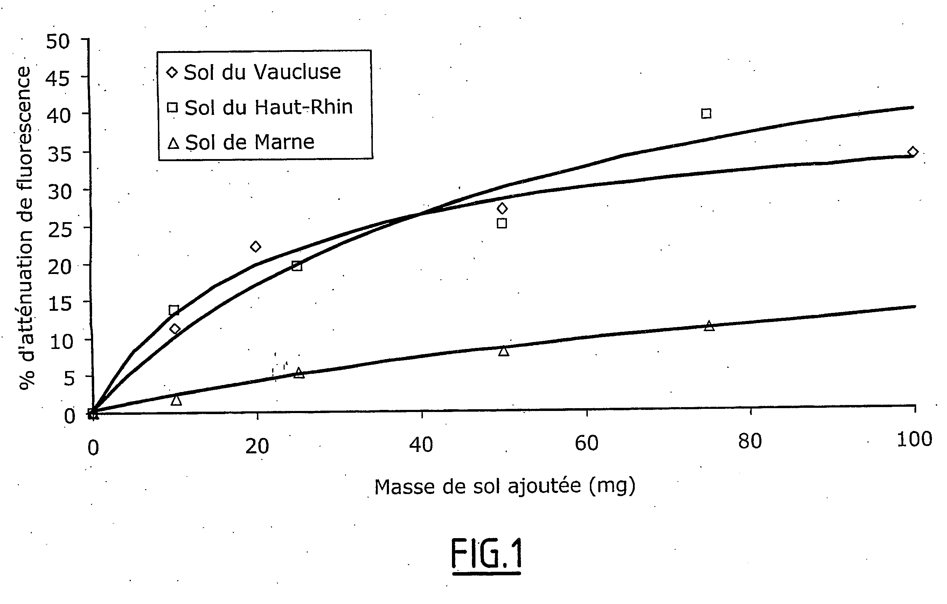

La figure 1 présente les résultats obtenus avec différents sols. L'échantillon testé est un échantillon aqueux de matière organique provenant d'un sol de surface (0-30 cm) d'un champ non cultivé de la région méditerranéenne obtenu par filtration à 0,45 µm et centrifugation (5 minutes à 5000 rpm). Les points correspondent aux résultats expérimentaux et les courbes ont été établies suivant un modèle affinité/saturation (Michaelis-Menten). Par exemple, les affinités de l'échantillon de sol obtenues pour les sols de la figure 1 sont : 20,8 mg pour le sol du Vaucluse, 50,9 mg pour le sol du Haut-Rhin et 139,3 mg pour le sol de la Marne.

### Réactivité avec des exoenzymes :

Le composant du sol mis en contact avec l'échantillon à tester peut être une exoenzyme, c'est-à-dire une enzyme utilisée par les microorganismes de manière extracellulaire pour rompre certaines molécules de haut poids moléculaire, telle que la cellulose, non assimilables sous forme entière. Les exoenzymes peuvent être notamment des hydrolases ou des oxydoréductases, enzymes impliquées dans le processus d'humification, c'est-à-dire d'allongement des chaînes carbonées polyphénols. Il peut s'agir par exemple de la β-glucosidase ou la laccase.

La mesure directe de l'activité enzymatique ne pouvant se faire par détection par fluorescence, on analyse l'inhibition compétitive par la matière organique de l'échantillon d'une réaction entre une exoenzyme et un substrat fluorescent de l'exoenzyme. Un substrat fluorescent de référence est ainsi ajouté à l'exoenzyme afin de réaliser une mesure indirecte, basée sur l'inhibition compétitive opérée par les substrats contenus dans l'échantillon de matières organiques sur la réaction entre le substrat artificiel et l'enzyme. Comme substrat, on choisit de préférence un substrat spécifique de l'enzyme ayant une affinité (constante de Michaelis-Menten, K_{M}) pour celle-ci d'un ordre de grandeur similaire à l'affinité d'un substrat naturel de l'enzyme potentiellement présent dans l'échantillon. Le rapport des affinités (rapport des K_{M} des deux substrats pour l'enzyme) détermine le rapport des concentrations utilisées dans le test. La détermination de ces concentrations peut faire l'objet d'une étude préliminaire, par exemple en utilisant le cellobiose pour la β-glucosidase.

Dans un exemple hors invention, la réaction d'inhibition compétitive prend place dans les puits de la microplaque en faisant réagir 90 µl de substrat artificiel fluorescent (le resorufin-βD-glucopyranoside) à une concentration déterminée selon les affinités (environ 10⁻⁵ M dans MES), avec 90 µl d'enzyme (1 unité/ml dans le tampon MES, pH 6,3 dans KNO₃ 0,1 M) et 90 µl d'échantillon contenant la matière organique à tester. De plus, trois puits contrôle sont réalisés : l'un contenant 90 µl de substrat artificiel tamponné, 90 µl de tampon et 90 µl d'eau pour vérifier la non-dégradation du substrat en absence d'enzyme ; le second contenant 90 µl de substrat, 90 µl d'enzyme et 90 µl d'eau afin d'obtenir une valeur référence de la réaction enzymatique, sans inhibition compétitive et le troisième contenant 90 µl de substrat, 90 µl d'échantillon et 90 µl de tampon afin de vérifier la non-dégradation du substrat par les matières organiques de l'échantillon. Ces réactions sont réalisées en duplicat.

Pour l'ensemble des huit puits, on examine la cinétique pour l'apparition de produit fluorescent (les longueurs d'ondes d'excitation et d'émission de fluorescence sont données dans la notice commerciale) dérivé du substrat dégradé par l'enzyme. Plus l'échantillon de matière organique réagit avec l'enzyme, plus la réaction avec le substrat artificiel est inhibée et la vitesse d'apparition de fluorescence mesurée (intensité de fluorescence en fonction du temps) diminue.

L'interprétation des résultats peut se faire de deux manières différentes. Tout d'abord, elle peut être réalisée par simple calcul de l'inhibition de la réaction en présence de l'échantillon (compétiteur) comparée à celle obtenue en absence d'échantillon : moins de 20% (réactivité avec l'exoenzyme faible), de 20 à 60% (réactivité moyenne) et plus de 60% (réactivité forte). Elle peut également être réalisée par comparaison des mesures de fluorescence de l'échantillon par rapport à la fluorescence d'une gamme étalon de concentrations d'un second substrat connu non fluorogénique (par exemple, le cellobiose) de l'exoenzyme qui génère une compétition et donc une réponse identique. Le résultat de la compétition exercée par l'échantillon est lu en terme de concentration de substrat et exprimé en équivalent substrat. La lecture des résultats bruts en terme de vitesse de réaction enzymatique pour différentes concentrations de substrat permet de calculer l'affinité de l'échantillon de matière organique envers l'enzyme par l'équation de Michaelis-Menten.

A noter que les longueurs d'onde d'excitation et d'émission des substrats enzymatiques artificiels et des produits de leurs réactions enzymatiques sont en général comprises dans le spectre visible et les problèmes d'auto-fluorescence (de la microplaque) sont donc minimisés.

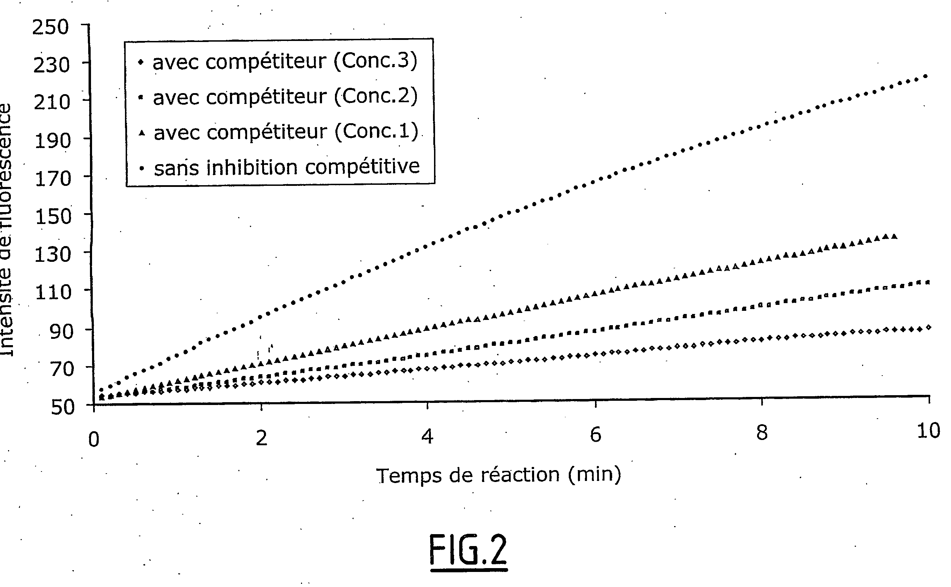

La figure 2 présente un exemple de gamme étalon obtenu avec une enzyme (la cellobiase extraite *d'Aspergillus niger*). L'échantillon est une solution de resorufin-βD-glucopyranoside, le substrat fluorescent, contenant différents ajouts de cellobiose, qui est le compétiteur. La figure montre la cinétique d'apparition de fluorescence correspondant à la formation du produit de la réaction entre le substrat fluorescent et l'enzyme, en absence de compétiteur et en présence de celui-ci à différentes concentrations croissantes. Le compétiteur est la cellobiose, substrat naturel de l'enzyme, retrouvé dans les échantillons de sol.

### Réactivité avec des microorganismes :

Le composant du sol peut être un microorganisme, en particulier une bactérie de préférence choisie parmi *Pseudomonas fluorescens, Azospirillum* 4B, *Acetogenium, Fusarium,* etc. Dans le cas de microorganismes anaérobies, il est préférable de placer une ou quelques gouttes de paraffine sur le puits rempli de la microplaque, afin de stopper le transfert d'oxygène et se placer ainsi en conditions anaérobies.

On analyse alors la variation de fluorescence en présence d'un révélateur rédox fluorescent sensible au catabolisme de la matière organique de l'échantillon.

Ce type de révélateur est connu de l'homme du métier. Il permet de mesurer l'évolution du couple NADH/NAD⁺ (Abe et al., 2002 ; Chen et al., 2000 ; Gloeckner et al., 2001).

Le révélateur rédox peut intervenir dans la chaîne de transport d'électron, et est réduit par couplage avec NAD⁺/NADH, révélant ainsi le phénomène de respiration.

Le révélateur peut être le 2,3,4,5,6-pentafluorodihydrotetraméthylrosamine (Redox Sensor™ Red CC-1, Chen et al., 2000), Le MTT (bromure de 3-(4,5-diméthylthiazol-2-yl)-2,5-diphényltétrazolium) est un composé jaune soluble dans l'eau qui, une fois réduit, forme un sel de formazan violet insoluble en milieu aqueux. On reprend les cristaux de formazan avec du dimethyl sulfoxyde (DMSO) pour pouvoir lire la densité optique.

Un autre révélateur est la resazurine (Atamarblue™, voir brevet U.S. 5,501,959). Celui-ci présente le double avantage du changement de couleur mais aussi de la formation d'un produit fluorescent. Ainsi ce produit peut aussi bien être utilisé en colorimétrie qu'en spectrofluorimétrie (Abe et al., 2002 ; Gloeckner et al., 2001 ; Kuda et al. ; 2003).

De plus, cet indicateur possède un potentiel rédox supérieur à celui du MTT, et se situe entre la réduction d'oxygène et du cytochrome oxydase, donc la chaîne respiratoire s'effectue jusqu'au bout sans risque d'interruption.

Le changement de couleur entre la forme oxydée non fluorescente, bleue et la forme réduite fluorescente, violette est marqué et. s'observe à l'oeil nu comme en spectroscopie.

Dans un exemple de réalisation, la réaction ne se fait plus directement en puits, mais passe par une étape en bouteille ambrée (20 ml). Le but de ce changement de protocole est de ne pas limiter l'apport d'oxygène lors de la respiration. Les solutions de substrat (glucose), révélateur rédox (Alamarblue) et de *Pseudomonas,* sont ajoutées dans les mêmes rapports.

La souche de microorganisme est conservée dans le milieu de croissance, avant d'être utilisée, par exemple sous la forme d'une solution saline comprenant de l'ordre de 10⁷ à 10⁸ bactéries/ml.

La solution saline peut être un milieu salin minéral (MSM) dépourvu dé carbone organique (1 litre de MSM a la composition suivante, en grammes : NaH₂PO₄.H₂O 0,883 ; K₂HPO₄ 2,257 ; (NH₄)₂SO₄ 1,1 ; MgSO₄.7H₂O 0,096 ; NaNO₃ 1,001 ; À cette solution est ajoutée 1 ml/l de la solution suivante de composition, en mg/L : Co(NO₂).6H₂O 291; AlK(SO₄)₂.12H₂O 474; CuSO₄ 160 ; ZnSO₄.7H₂O 288; FeSO₄.7H₂O 2780 ; MnSO₄.H₂O 1690 ; Na₂MoO₄:2H₂O 482 ; Ca(NO₃)₂.4H₂O 2362). La mesure de l'activité est ici réalisée à l'aide d'un révélateur rédox fluorescent, l'Alamar Blue (solution commerciale) qui change de spéciation et donc de coordonnées de fluorescence lorsque des électrons sont échangés (chaîne respiratoire) lors de la prise en charge d'un substrat par le microorganisme.

La réaction prend place dans quatre puits de la microplaque : le premier contient 90 µl de révélateur rédox (30% de la solution commerciale) et 180 µl de MSM afin de vérifier sa non-dégradation au cours de la mesure ; le second contient 90 µl de révélateur rédox, 90 µl d'échantillon et 90 µl de MSM pour vérifier l'absence de réaction entre les deux ; le troisième contient 90 µl de révélateur rédox, 90 µl de solution de microorganismes et 90 µl de MSM pour vérifier le niveau du bruit de fond de métabolisme des microorganismes en l'absence de substrat organique. Enfin, le dernier contient 90 µl de chaque réactif (microorganismes, révélateur et échantillon) et sert de mesure de l'activité. Le dispositif des quatre puits est répété une fois.

Les huit puits sont suivis au cours de l'incubation à 37°C pendant 48 à 72 heures, à une fréquence choisie (ex. : 2, 4, 6, 8, 12, 24, 36, 48, 72). A chaque temps, une lecture des huit puits est effectuée et l'intensité de fluorescence est comparée aux contrôles pour obtenir une activité.

L'interprétation peut se faire de deux manières différentes. Elle peut être réalisée par simple calcul de l'inhibition de la réaction en présence de l'échantillon comparée à celle obtenue pour le témoin ; moins de 20% (réactivité avec le microorganisme faible), de 20 à 60% (réactivité moyenne) et plus de 60% (réactivité forte). Elle peut également être réalisée par comparaison des mesures de fluorescence de l'échantillon par rapport à une gamme de concentrations d'un substrat facilement dégradable, tel le glucose. Le résultat de l'échantillon est lu en terme de concentration de glucose et exprimé en équivalent glucose.

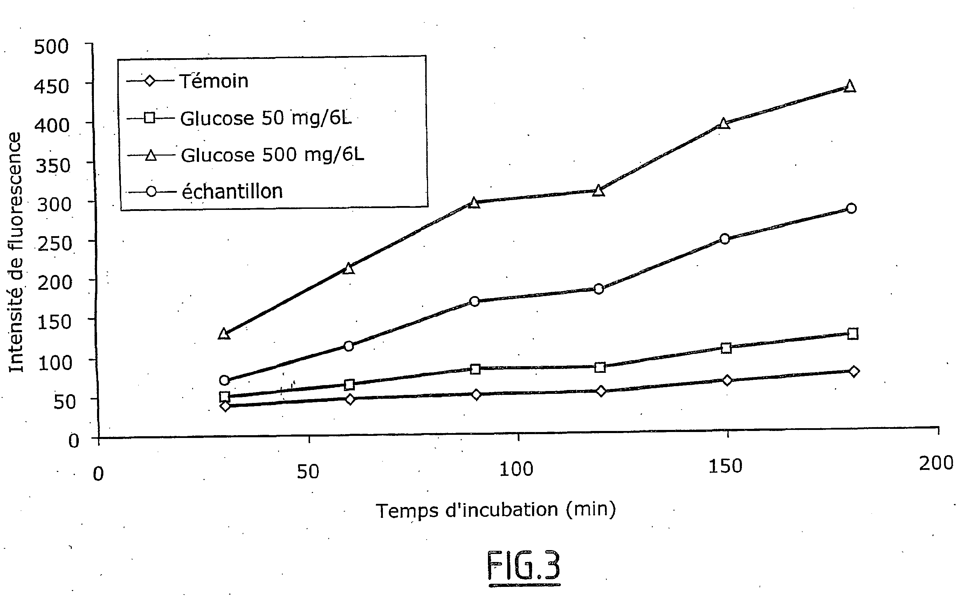

La figure 3 présente les résultats obtenus avec une souche AK15 de *Pseudomonas fluorescens,* le substrat étant le glucose et l'échantillon aqueux de matière organique provenant d'un sol de surface (0-30 cm) d'un champ non cultivé de la région méditerranéenne. La figure montre la cinétique d'apparition de fluorescence correspondant à l'accumulation du révélateur rédox sous sa forme réduite. Le témoin correspond à la souche bactérienne en présence du révélateur, mais sans glucose. L'échantillon aqueux de matière organique, qui présente un contenu en carbone de 226 mg C/L, a une activité catabolique bactérienne, déduite de la courbe, évaluée à 20 mg C/L (100 mg Glu/L ↔ 6,7 mg C/L), ce qui correspond à 9% du contenu total.

Ce type de mesure donne une indication de la façon dont un échantillon aqueux de matière organique peut être dégradé par les bactéries du sol.

### Réactivité avec des cations métalliques :

Le composant du sol peut être un cation métallique, par exemple choisi parmi par Cu²⁺, Ni²⁺, Cr³⁺ et le Cd²⁺.

Le cation métallique est en solution acide (par exemple à pH 2).

Selon un exemple hors invention, on analyse l'extinction (« quenching ») de la fluorescence de la matière organique complexée au cation métallique présent à concentrations croissantes (Ryan et Weber, 1982).

Selon un exemple hors invention, la réaction de complexation des métaux par les matières organiques de l'échantillon se fait dans les puits de la microplaque. 10 µl de solution de cation(s) métallique(s) à concentrations croissantes (0 à 2,5.10⁻³ M à pH 3 à 4 dans HCl 0.1 M) sont ajoutés à 200 µl d'échantillons et 50 µl de tampon (N-méthyl-piperazine 0,1M dans KNO₃ 0,1 M, pH 9,3). Le temps de réaction est de deux minutes après mélange.

Les longueurs d'onde utilisées pour la détection correspondent à celles de la matière organique (λₑₓ 330 nm et λₑₘ 440 nm). La complexation des cations par la matière organique génère une diminution de l'intensité de fluorescence.

L'interprétation des résultats peut se faire de deux manières, en fonction de l'utilisation ou non d'une analyse mathématique. Premièrement, l'interprétation peut être réalisée par simple calcul du pourcentage de l'échantillon complexé, et être ainsi comparé à d'autres échantillons ou fournir un ordre de grandeur de l'interaction entre l'échantillon et le cation métallique. La lecture simple peut se faire sur la concentration de cation métallique à ajouter pour atténuer le signal de fluorescence de 50% (équivalent LD50) avec une grille de lecture en trois niveaux : moins de 20 µM (forte complexation), entre 20 et 100 µM (complexation moyenne), plus de 100 µM (faible complexation). Deuxièmement, l'interprétation des résultats peut être réalisée à l'aide du modèle simple affinité/saturation (de type Michaelis-Menten ou Langmuir) qui fournit un paramètre quantitatif : l'affinité entre les matières organiques solubles et le cation métallique.

Les résultats bruts sont reportés en terme d'intensité de fluorescence relative (intensité de fluorescence mesurée, rapportée à l'intensité de fluorescence du puits avec l'échantillon seul) en fonction de la concentration en cation métallique. La régression non-linéaire entre la courbe obtenue et le modèle affinité-saturation, effectuée à l'aide du logiciel libre WinReg (http://www.unilim.fr/pages_perso/jean.debord/winreg/winreg1.htm) donne les paramètres de complexation : la constante d'équilibre (Kc) et la quantité totale de métal complexé à saturation.

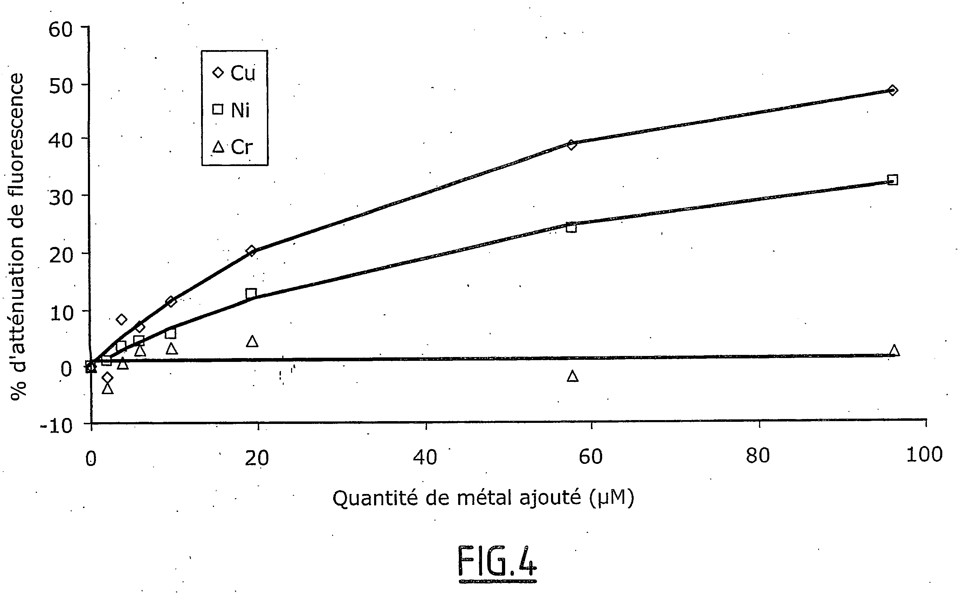

La figure 4 présente les résultats obtenus avec plusieurs cations (Cu²⁺, Ni²⁺, Cr³⁺). La figure montre l'ampleur de l'atténuation de fluorescence d'un échantillon de sol observée lors d'ajouts croissants de concentrations des cations métalliques. Les points correspondent aux résultats expérimentaux et les courbes ont été établies suivant un modèle affinité/saturation. Les affinités de l'échantillon de matière organique provenant d'un extrait aqueux de sol agricole du Vaucluse obtenues pour les cations de la figure 4 sont : 51,4 µM pour le Cu²⁺, 70,0 µM pour le Ni²⁺ et une valeur infinie pour le Cr³⁺ car aucune complexation n'est observée. L'affinité représente la concentration de cation métallique à rajouter pour obtenir la demi-saturation, donc plus la valeur est faible, plus l'affinité est forte.

Selon un autre exemple hors invention, on analyse l'augmentation de la fluorescence de la matière organique liée à l'inhibition compétitive de la complexation de la matière organique au cation métallique par un ligand du cation métallique d'affinité connue pour le cation.

Selon un exemple hors invention, on mesure l'émission de fluorescence du mélange d'une quantité donnée de matière organique et de cation métallique et on la compare aux intensités de fluorescence mesurées en présence en outre d'un ligand du cation métallique ou de plusieurs ligands du cation métallique, pris séparément, l'affinité des ligands pour le cation métallique étant connue. Les ligands sont de préférence ajoutés en une quantité donnée de manière à ce que l'augmentation de fluorescence en présence de ligand, par rapport au contrôle sans ligand, soit corrélée à l'affinité du ligand pour le cation métallique.

Les résultats peuvent être reportés en terme d'intensité de fluorescence relative (intensité de fluorescence mesurée en présence de ligand rapportée à l'intensité de fluorescence mesurée en l'absence de ligand) en fonction de l'affinité des ligands du cation métallique utilisés.

Des exemples de ligands de cation métallique incluent l'EDTA et de dérivés de l'EDTA, différents acides carboxyliques, les dérivés du phénol, ou encore des amines.

### Réactivité avec des micropolluants organiques :

Le composant du sol peut être un micropolluant organique, tel qu'un pesticide (par exemple l'atrazine) ou un hydrocarbure aromatique polycyclique (HAP), tel que le phénanthrène (les HAP étant les polluants systématiques du sol des anciennes usines à gaz et des cokeries).

Selon un exemple hors invention, on analyse la variation de la fluorescence de la matière organique en utilisant un micropolluant organique, dont on fait varier la concentration.

Dans un exemple hors invention, la réaction de complexation des micropolluants par les matières organiques de l'échantillon se fait dans les puits de la microplaque. 10 µl d'une solution de micropolluant de concentrations croissantes (0 à 10⁻⁴ M) sont ajoutés à 200 µl d'échantillons et 50 µl de tampon (N-methyl-piperazine 0,1 M, pH 9,3 dans KNO₃ 0.1 M). Le temps de réaction est de deux minutes après mélange.

Les longueurs d'onde utilisées pour la détection correspondent à celles de la matière organique (λₑₓ 330 nm et λₑₘ 440 nm). La complexation des polluants par la matière organique génère une diminution de l'intensité de fluorescence.

Les résultats bruts sont reportés en terme d'intensité de fluorescence relative (intensité de fluorescence mesurée et rapportée à l'intensité de fluorescence du puits avec l'échantillon seul) en fonction de la concentration en micropolluant. La régression non-linéaire entre la courbe obtenue et lé modèle affinité-saturation donne les paramètres de complexation : la constante d'équilibre (K_{C}) et la quantité totale de micropolluant complexé à saturation.

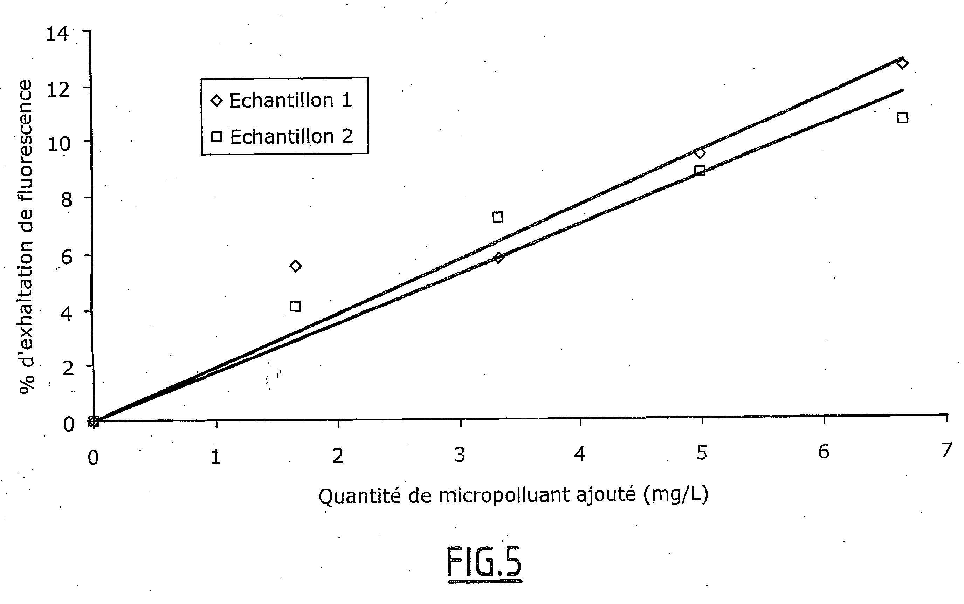

La figure 5 présente les résultats obtenus avec un polluant (l'isoproturon). La figure montre l'ampleur de l'exhaltation de fluorescence de deux extraits aqueux du même sol (correspondant pour le premier au sol prélevé en surface (0-30 cm) et pour le second prélevé à une profondeur comprise entre 30-60 cm) observée lors d'ajouts de concentrations croissantes d'un micropolluant organique non-fluorescent (isoproturon). Les points représentent les résultats expérimentaux et les droites représentent les approximations linéaires de ces points.

L'interprétation peut se faire selon l'ampleur de variation de l'intensité de fluorescence (exhaltation ou quenching) observée lors d'une augmentation d'un facteur 2 de la concentration en micropolluant organique. Ceci correspond à un paramètre généralement connu sous le terme Q₂. Le Q₂ mesuré pour les échantillons 1 et 2 (Figure 5) est respectivement de 3,3 et 3,1% calculé sur l'étendue des concentrations d'isoproturon testées. Q₂ inférieurs à 5% (réactivité faible avec le micropolluant organique), entre 5 et 25% (réactivité moyenne), et au-delà de 25% (réactivité forte).

Selon un autre exemple hors invention, on analyse l'extinction (« quenching ») de la fluorescence d'un micropolluant organique fluorescent comme proposé par Fang et al., 1998. On peut citer comme micropolluant organique fluorescent les composés de la famille des hydrocarbures aromatiques polycycliques (HAP) ou certains pesticides. Ce mode de réalisation permet de s'affranchir de la mesure de fluorescence de la matière organique de l'échantillon, si désiré.

### Kit :

La demande décrit également un kit pour la mise en oeuvre du procédé tel que défini ci-dessus, comprenant une microplaque et au moins un récipient contenant un échantillon de sol sec ou un composant d'un sol.

L'invention a également pour objet un kit pour la mise en oeuvre du procédé selon l'invention, comprenant une microplaque, au moins un récipient contenant un composant d'un sol, ledit composant d'un sol étant un microorganisme, et un révélateur rédox fluorescent sensible au catabolisme de la matière organique d'un échantillon aqueux de matière organique à tester.

Le kit peut ainsi comprendre un récipient contenant un premier échantillon de sol sec ou un premier composant d'un sol, et au moins un récipient contenant un deuxième échantillon de sol sec ou un deuxième composant d'un sol.

Des solutions tampons, les substrats enzymatiques et/ou les révélateurs rédox peuvent être ajoutés.

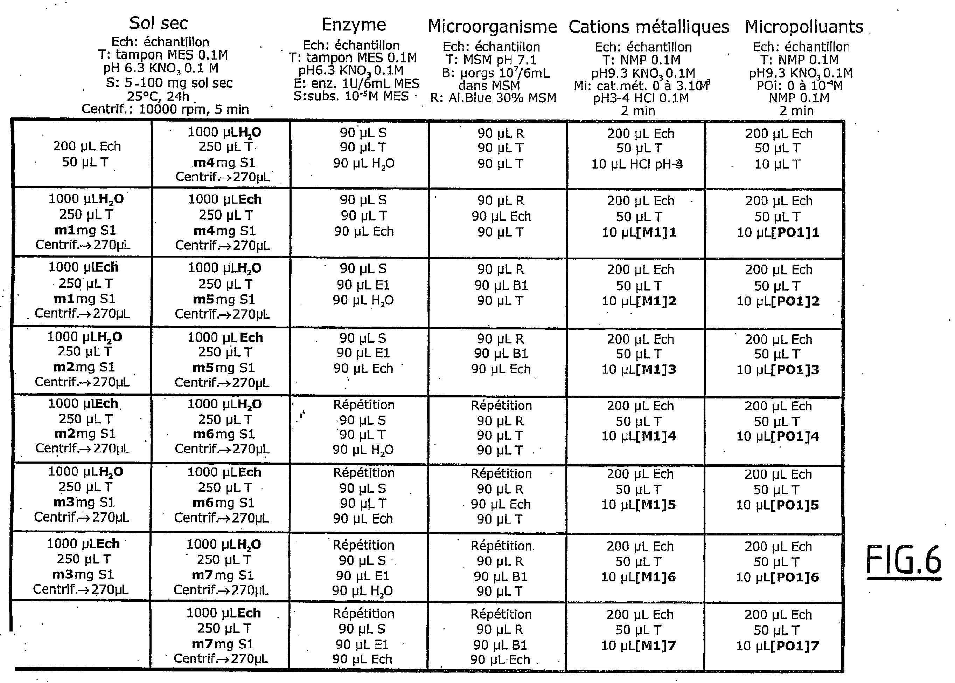

La figure 6 est un schéma général de la microplaque utilisée dans les exemples ci-dessus : MES : acide 2[N-Morpholino]éthanesulfonique ; MSM : milieu salin minéral ; µorgs : microorganismes ; NMP : N-methylpyperazine ; µPO : micropolluant organique ; POi : i^{ème} concentration du micropolluant organique.

### BIBLIOGRAPHIE

- Abe et al. (2002) Neuroscienceletters, 326(3) : p. 179-182
- Chen et al. (2000) Free Radical Biology and Medicine 28(8) : p. 1266-1278
- Fang et al. (1998) Analytica Chimica 373 139-151
- Gloeckner et al. (2001) Journal of immunological methods, 252(1-2) :p. 131-138
- Ryan et Weber, (1982) Environ. Sci. Technol. 16, 866-872

## Revendications

1. Procédé de détermination de la réactivité biogéochimique d'un échantillon aqueux de matière organique, dans lequel le devenir de ladite matière organique dans un sol, est évalué, comprenant :
a. le mélange de l'échantillon avec un composant d'un sol avec lequel l'échantillon est susceptible de réagir, ledit composant d'un sol étant un microorganisme; et
b. l'analyse en microplaque de la fluorescence émise par ledit mélange en présence d'un révélateur rédox fluorescent sensible au catabolisme de la matière organique de l'échantillon;
une variation de fluorescence, par rapport à la fluorescence émise par le composant du sol en l'absence de l'échantillon ou par l'échantillon en l'absence du composant du sol, étant indicatrice de la capacité de l'échantillon à être dégradé par un microorganisme du sol.

2. Procédé selon la revendication 1, sous la forme d'un test multiformat, comprenant le mélange de l'échantillon avec plusieurs composants du sol pris séparément, et l'analyse de la fluorescence sur la même microplaque.

3. Procédé selon la revendication 1, dans lequel le composant du sol est un microorganisme choisi parmi les bactéries ou les champignons.

4. Procédé selon la revendication 1, dans lequel le composant du sol est un microorganisme choisi parmi *Pseudomonas fluorescens, Azospirillum* 4B, *Acetogenium, Fusarium.*

5. Procédé selon la revendication 1, dans lequel le composant du sol est un microorganisme anaérobie.

6. Procédé selon la revendication 5, dans lequel un ou deux gouttes de paraffine sont placées sur le puits rempli de la microplaque analysée à l'étape b) afin de stopper le transfert d'oxygène et de se placer ainsi en conditions anaérobies.

7. Procédé selon l'une quelconque des revendications 1 à 6, dans lequel le révélateur est le 2,3,4,5,6-pentafluorodihydrotetraméthylrosamine, ou la resazurine.

8. Procédé selon l'une des revendications 1 à 7, dans lequel l'échantillon aqueux de matière organique est choisi parmi des boues de stations d'épuration, des résidus ou effluents agro-industriels, des eaux de lacs, de sols, de rivières, et des eaux marines ou souterraines.

9. Procédé selon l'une quelconque des revendications 1 à 8, dans lequel l'échantillon aqueux de matière organique n'est pas constitué d'acide fulvique.

10. Procédé selon l'une des revendications 1 à 9, dans lequel un puits de la microplaque contient un volume inférieur à environ 1 ml dudit mélange.

11. Kit pour la mise en oeuvre du procédé selon l'une des revendications 1 à 10, comprenant une microplaque, au moins un récipient contenant un composant d'un sol, ledit composant d'un sol étant un microorganisme, et un révélateur rédox fluorescent sensible au catabolisme de la matière organique d'un échantillon aqueux de matière organique à tester.

12. Kit selon la revendication 11, comprenant au moins un récipient contenant un premier composant d'un sol, ledit premier composant d'un sol étant un microorganisme, et au moins un récipient contenant un échantillon de sol sec ou un deuxième composant d'un sol.

## Patentansprüche

1. Verfahren zur Bestimmung der biogeochemischen Reaktivität einer wässrigen Probe aus einem organischen Stoff, bei dem die Entwicklung des organischen Stoffs in einem Boden ausgewertet wird, umfassend:
a. die Mischung der Probe mit einer Komponente des Bodens, mit der die Probe geeignet ist, zu reagieren, wobei die Komponente des Bodens ein Mikroorganismus ist; und
b. die Analyse in einer Mikrotiterplatte der Fluoreszenz, die von der Mischung in Anwesenheit eines fluoreszierenden Redoxentwicklers emittiert wird, der für den Katabolismus des organischen Materials der Probe empfindlich ist;
wobei eine Änderung der Fluoreszenz in Bezug auf die Fluoreszenz, die von der Komponente des Bodens in Abwesenheit der Probe oder von der Probe in Abwesenheit der Komponente des Bodens emittiert wird, anzeigend ist für die Kapazität der Probe, durch einen Mikroorganismus des Bodens abgebaut zu werden.

2. Verfahren nach Anspruch 1 in Form eines Multiformaltests, die Mischung der Probe mit mehreren getrennt genommenen Komponenten des Bodens und die Analyse der Fluoreszenz auf derselben Mikrotiterplatte umfassend.

3. Verfahren nach Anspruch 1, bei dem die Komponente des Bodens ein Mikroorganismus ist, der ausgewählt ist aus den Bakterien oder den Pilzen.

4. Verfahren nach Anspruch 1, bei dem die Komponente des Bodens ein Mikroorganismus ist, der ausgewählt ist aus Pseudomas fluorescens, Azospirillum 4B, Acetogenium, Fusarium.

5. Verfahren nach Anspruch 1, bei dem die Komponente des Bodens ein anaerober Mikroorganismus ist.

6. Verfahren nach Anspruch 5, bei dem ein oder zwei Paraffintropfen auf die gefüllte Vertiefung der Mikrotiterplatte aufgegeben wird, die bei Schritt b) analysiert wird, um den Sauerstofftransfer zu stoppen und sich so in den anaeroben Zustand zu bringen.

7. Verfahren nach einem beliebigen der Ansprüche 1 bis 6, bei dem der Entwickler 2,3,4,5,6-Pentafluordihydrotetramethylrosamin oder Resazurin ist.

8. Verfahren nach einem der Ansprüche 1 bis 7, bei dem die wässrige Probe aus organischem Material ausgewählt ist aus Schlämmen der Kläranlagen, landwirtschaftlichen und industriellen Rückständen und Abwässern, Wässern von Seen, Böden, Flüssen und Meeresgewässern oder Grundwässern.

9. Verfahren nach einem beliebigen der Ansprüche 1 bis 8, bei dem die wässrige Probe aus organischem Stoff nicht aus Fulvosäure gebildet.

10. Verfahren nach einem der Ansprüche 1 bis 9, bei dem eine Vertiefung der Mikrotiterplatte ein Volumen kleiner als ungefähr 1 ml der Mischung enthält.

11. Kit zur Durchführung des Verfahrens nach einem der Ansprüche 1 bis 10, der eine Mikrotiterplatte, einen eine Komponente eines Bodens enthaltenden Behälter und einen fluoreszierenden Redoxentwickler umfasst, der empfindlich für den Katabolismus des organischen Stoffs einer zu testenden wässrigen Probe aus organischem Stoff ist, wobei die Komponente eines Bodens ein Mikroorganismus ist.

12. Kit nach Anspruch 11, der mindestens einen eine erste Komponente eines Bodens enthaltenden Behälter und mindestens einen eine Probe eines trockenen Bodens oder einer zweiten Komponente eines Bodens enthaltenden Behälter umfasst, wobei die erste Komponente eines Bodens ein Mikroorganismus ist.

## Claims

1. A method for determining the biogeochemical reactivity of an aqueous sample of organic matter, wherein the fate of said organic matter in a soil is evaluated, comprising:
a. The mixing of the sample with a component of a soil with which the sample is likely to react, said component of a soil being a microorganism; and
b. The microplate analysis of the fluorescence emitted by said mixture in the presence of a fluorescent redox developer sensitive to the catabolism of the organic matter of the sample;
a variation of fluorescence, relative to the fluorescence emitted by the soil component in the absence of the sample or by the sample in the absence of the soil component, indicating the capacity of the sample to be degraded by a soil microorganism.

2. The method as in claim 1, in the form of a multi-format test, comprising mixing of the sample with several soil components taken separately, and the analysis of the fluorescence on the same microplate.

3. The method as in claim 1, wherein the soil component is a microorganism chosen from among the bacteria or the fungi.

4. The method as in claim 1, wherein the soil component is a microorganism chosen from among *Pseudomonas fluorescens, Azospirillum* 4B, *Acetogenium, Fusarium.*

5. The method as in claim 1, wherein the soil component is an anaerobic microorganism.

6. The method as in claim 5, wherein one or two drops of paraffin are placed on the filled well of the microplate analysed in step b) in order to stop the transfer of oxygen and thus establish anaerobic conditions.

7. The method as in any one of claims 1 to 6, wherein the developer is 2,3,4,5,6-pentafluorodihydrotetramethylrosamine or resazurin.

8. The method as in any one of claims 1 to 7, wherein the aqueous sample of organic matter is chosen from among treatment plant sludges, agro-industrial residues or effluents, waters from lakes, soils, and rivers, and marine or underground waters.

9. The method as in any one of claims 1 to 8, wherein the aqueous sample of organic matter is not composed of fulvic acid.

10. The method as in any one of claims 1 to 9, wherein a well of the microplate contains a volume of less than approximately 1 ml of said mixture.

11. The kit for carrying out the method as in one of claims 1 to 10, comprising a microplate, at least one container containing a component of a soil, said component of a soil being a microorganism, and a fluorescent redox developer sensitive to the catabolism of the organic matter of an aqueous sample of the organic matter to be tested.

12. The kit as in claim 11, comprising at least one container containing a first component of a soil, said first component of a soil being a microorganism, and at least one container containing a sample of dry soil or a second component of a soil.
